# EUROPÄISCHE PATENTSCHRIFT

(11) **EP 0 852 779 B1**
(45) Veröffentlichungstag und Bekanntmachung des Hinweises auf die Patenterteilung: **18.07.2001**
(21) Anmeldenummer: 96945135.0
(22) Anmeldetag: 18.09.1996
(51) Int. Cl.: G08B 17/117, G01N 31/22

(54) **BIOSENSORSYSTEM ZUR MESSUNG VON DURCH SCHWELBRÄNDE VERURSACHTEN, ORGANISCHEN SPURENKOMPONENTEN**
BIOSENSOR SYSTEM FOR MEASURING LEVELS OF ORGANIC TRACE COMPONENTS PRODUCED BY SMOULDERING FIRES
SYSTEME DE BIOCAPTEUR POUR DETECTER LES COMPOSANTS ORGANIQUES TRACES PRODUITS PAR DES FEUX COUVANTS

(30) Priorität: 29.09.1995 DE 19536384
(43) Veröffentlichungstag der Anmeldung: 15.07.1998
(73) Patentinhaber: FORSCHUNGSZENTRUM JÜLICH GMBH, 52425 Jülich (DE)
(72) Erfinder: SCHÖNING, Michael, Josef, D-52428 Jülich (DE); SCHÜTZ, Stefan, D-35392 Giessen (DE); WEISSBECKER, Bernhard, D-63548 Gründau (DE); SCHWARZ, Axel, D-35396 Giessen (DE); KOHL, Claus-Dieter, D-35396 Giessen (DE); HUMMEL, Hans, E., D-35394 Giessen (DE); KORDOS, Peter, D-52428 Jülich (DE); LÜTH, Hans, D-52076 Aachen (DE)
(86) Internationale Anmeldenummer: DE9601799
(87) Internationale Veröffentlichungsnummer: WO9712218

(56) Entgegenhaltungen:
- EP-A- 0 545 547
- DE-A- 4 338 732
- DE-A- 4 403 152
- US-A- 5 308 763
- US-A- 5 331 310
- ANALYTICAL CHEMISTRY, Bd. 61, Nr. 8, 15.April 1989, Seiten 533A-542A, XP002030413 R. M. BUCH ET AL: "neuronal biosensors"
- TRAC, TRENDS IN ANALYTICAL CHEMISTRY, Bd. 14, Nr. 7, 1.August 1995, Seiten 300-303, XP000515015 DAMGAARD L R ET AL: "MICROSCALE BIOSENSORS FOR ENVIRONMENTAL MONITORING"
- NEC RESEARCH AND DEVELOPMENT, Nr. 99, 1.Oktober 1990, Seiten 1-8, XP000178470 JUN KIMURA ET AL: "DEVELOPMENT OF BIOSENSORS BASED ON ION SENSITIVE FIELD EFFECT TRANSISTORS"

## Beschreibung

Die Erfindung betrifft ein Biosensorsystem zur Messung von durch Schwelbrände verursachten, organischen Spurenkomponenten, gemäß dem Oberbegriff des Anspruchs 1.

Methoden zur Früherkennung von Bränden basieren am häufigsten auf optischen Meldern, die die Lichtstreuung von Aerosolen messen. Diese Methoden sind jedoch nur eingeschränkt zuverlässig. Rückläufig ist die Entwicklung von Ionisationsmeldern, die ein radioaktives Präparat nutzen, um mit einem empfindlichen Verstärker den Strom der ionisierten Aerosole zu messen. Hauptursache dafür ist die kostenintensive Handhabung der radioaktiven Präparate. Weiterhin werden Thermokameras genutzt, mit denen 0,1 K Temperaturerhöhung über eine große Fläche gemessen werden kann, z. B. für die Überwachung des Müllvorrates in Müllverbrennungsanlagen. Die Kosten sind jedoch erheblich, die Zuverlässigkeit ist umstritten.

In jüngster Zeit finden verstärkt Gassensoren auf der Basis von Halbleitern bzw. elektrochemischen Zellen Verwendung. Gemessen werden die Konzentrationen von CO, bei katalytisch aktiven Oberflächen H₂, oder Kohlenwasserstoffe. Die Nachweisempfindlichkeit liegt bei etwa 1 ppm, die Nachweissicherheit wird jedoch in unterschiedlichem Maße durch Hintergrundgase (z. B. Kfz-Abgase) beeinträchtigt (D. Kohl et al.: Gassensortechnik zur Erkennung von Schwelbränden., in "Brandmeldeanlagen", VdS-Fachtagung 1993).

Nachteilig bei den bekannten Meßverfahren, insbesondere auf dem Gebiet der Brandfrüherkennung, sind vor allem die mangelnde Selektivität sowie Sensitivität für die warnauslösenden Substanzen. Zur Erhöhung der Nachweissicherheit sind deshalb Sensoren notwendig, die die brandspezifische Erkennung von organischen Spurenstoffen mit höherer Sensitivität ermöglichen.

In US 5,331,310 wird ein CO-Brandsensor beschrieben, der auf amperometrische Weise sehr empfindlich CO nachweisen kann.

Aus DE 43 38 732 ist ein Biosensor bekannt, der durch Einbringen von speziell ausgesuchten organischen Molekülen (Rezeptormoleküle) in einen Polymerfilm, verbunden mit einem organischen Halbleiter, hochselektiv die zu den Rezeptoren passenden Liganden identifizieren kann.

In einem Übersichtsartikel von R. M. Buch und G. A. Rechnitz, Analytical Chemistry, Vol. 61(8) 1989, Seiten 533-541, wird über einen Versuchsaufbau berichtet, bei dem eine Krabbenantenne als Sensor für chemische Substanzen in wäßrigen Medien eingesetzt wird.

Es ist deshalb Aufgabe der Erfindung, einen Biosensor zu schaffen, bei dem eine hohe Sensitivität und Selektivität sowie geringe Störanfälligkeit und portabler Einsatz für Feldmessungen zur Detektion brandspezifischer Emissionen, z. B. multifunktioneller Phenole, Alkene oder Terpene, erzielt werden kann.

Die Aufgabe wird gelöst durch einen Biosensor gemäß der Gesamtheit der Merkmale nach Anspruch 1. Weitere zweckmäßige oder vorteilhafte Ausführungsformen finden sich in den auf den Anspruch 1 rückbezogenen Unteransprüchen.

Das entwickelte Biosensorsystem zur Brandfrüherkennung basiert auf dem Nachweis von organischen Verbindungen, die in der frühen Phase eines Brandes freigesetzt werden (Detektion von "Brandgeruch").

Das entwickelte Biosensorsystem besteht aus einer Meßluftzuführung, einer Biokomponente, einer Halbleiterkomponente, einer Probenzellenanordnung sowie einer Signal- und Meßwertverarbeitung. Die Meßluftzuführung ermöglicht die definierte Probennahme für die Biokomponente. Die Biokomponente besteht aus einem intakten Chemorezeptor, z.B. der Insektenantenne eines Borkenkäfers. Dabei kann der Chemorezeptor gleichstrommäßig oder auch wechselstrommäßig (z.B kapazitiv oder Influenz) mit der Halbleiterkomponente verbunden sein.

Der Erkennungsmechanismus erfolgt nach Durchtritt durch die Poren der schützenden Chitin-Cuticula in die Rezeptorlymphe dieser Insektenantenne durch hochspezifische (Pheromene Binding Protein = PBP) oder gruppenspezifische (General Odorant Binding Protein = GOBP) Proteine, die ihrerseits an die membranbeständigen Rezeptoren oder Dendriten binden und dadurch die Depolarisation der Neuronenmembran bewirken. Diese Depolarisationen können mit hochohmigen Halbleiter-Verstärkern, z.B. Feldeffekttransistoren, meßbar gemacht werden. Mittels einer geeigneten Signal- und Meßwertverarbeitung ist eine Quantifizierung der auslösenden Substanz über mehrere Größenordnungen möglich.

Die erforderliche hohe Selektivität und Sensitivität des Biosensorsystems kann durch die geeignete Auswahl der Biokomponente, - Art, Zucht und Präparation der verwendeten Insektenantenne, z.B. aus der Familie der Borkenkäfer - gewährleistet werden. Aufgrund der Miniaturisierbarkeit der Schnittstelle zwischen intaktem Chemorezeptor und Halbleiterkomponente kann die Betriebssicherheit des Biosensorsystems erhöht und gleichzeitig Volumen-, Gewicht- und Energiebedarf verringert werden. Durch die Kombination eines solchen Biosensorsystems mit handelsüblichen Gassensoren für typische Verbrennungsgase kann eine erhöhte Detektionssicherheit erreicht werden.

Die Erfindung ist im weiteren an Hand von Figuren und Ausführungsbeispiel näher erläutert. Es zeigen:
- Fig. 1:: Biosensorsystem;
- Fig. 2:: Vergrößerte Darstellung des Systems nach Figur 1 im Bereich des Biosensors;

### Ausführungsbeispiel

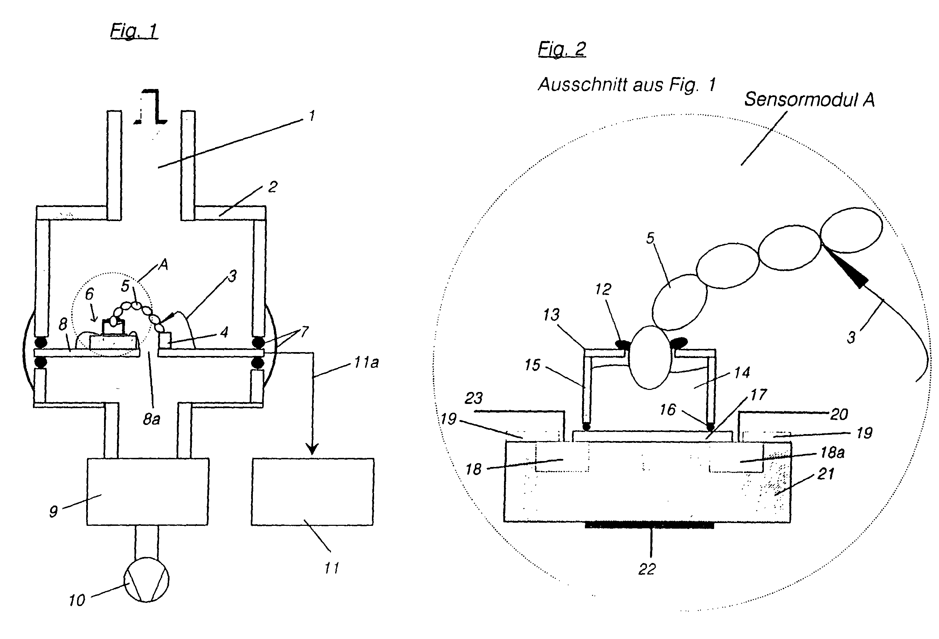

In Figur 1 ist das entwickelte Biosensorsystem am Beispiel eines intakten Chemorezeptors eines Borkenkäfers dargestellt. Ein solches System kann Einsatz finden, z.B. im Bereich der Brandfrüherkennung.

Die Meßluftzuführung 1 aus einem festen oder mittels Kugelschliff beweglichen Rohr aus z.B. Glas, Teflon oder Edelstahl mit einem Durchmesser im Bereich von bis zu 100 mm, insbesondere zwischen 5 und 15 mm, mündet in eine Probenzellenanordnung 2 aus z.B. Glas, Teflon oder Edelstahl. Die Probenzellenanordnung ermöglicht einen einfachen und schnellen Wechsel bzw. Austausch des Sensormoduls 5,6 und gewährt die für den portablen Einsatz erforderliche, mechanische Stabilität.

Über geeignete mikromechanische Arretier- bzw. Haltevorrichtungen 7 wird das Sensormodul 5,6 und folglich die Biokomponente stabil und geschützt positioniert. Eine weitere Erhöhung der elektrischen Störsicherheit kann z.B. durch das Einbringen des Sensormoduls in einen miniaturisierten, auf einem stabilen elektrischen Potential befindlichen Faradaykäfig erzielt werden.

Das Sensormodul 5,6 wird mit einem festen Träger 8, z.B. nit einem mit einer Öffnung 8a für die durchströmende Luft zur Beprobung der Biokomponente versehenen Leiterplattensubstrat 8, verbunden. Zur weiteren mechanischen Stabilisierung der Antenne 5 ist jenseits der Öffnung 8a eine elektrisch isolierende Stütze 4 vorgesehen.

Beispielsweise über ein als Pulsationsdämpfer wirkendes Gefäß 9 wird die Meßluft von einer Pumpe 10 mit einem der Probenzellenanordnung angemessenen Durchsatz von beispielsweise 0 - 5 l/min, insbesondere 0,5 - 1,5 l/min abgesaugt. Anstelle der Pumpenanordnung (9, 10) kann im Falle der Detektion von Brandgasen auch die natürliche Luftbewegung bzw. -strömung oder - diffusion, hervorgerufen durch den Brandherd, zum Transport der Brandemissionen vom Brandherd zum Biosensorsystem ausgenutzt werden.

Die vom Sensormodul 5,6 erhaltenen Meßsignale werden über vorzugsweise abgeschirmte Zuleitungen lla an eine Signal- und Meßverarbeitungseinheit 11 weitergeleitet. Das resultierende Signal hängt dabei direkt von der Intensität des detektierten "Brandgeruchs", d.h. von Art und Stärke der Brandemission ab.

Der in Figur 2 dargestellte Biosensor 5,6 setzt sich aus einer Halbleiterkomponente, vorzugsweise einem Feldeffekttransistor (FET, z.B. einem MOSFET) einer Insektenantenne 5, z.B. dem intakten Chemorezeptor 5 eines Borkenkäfers, sowie einer Verbindungseinheit, die den elektrischen Kontakt zwischen der Antenne und dem FET bewirkt, zusammen.

Der intakte Chemorezeptor 5 ist am einen Ende über eine Ableitelektrode 3 aus z.B. Glas, Metall, vorzugsweise Ag, Pt oder Ag/AgCl kontaktiert. Am anderen Ende ist sie über einen Flüssig- oder Festkontakt 14, vorzugsweise eine Elektrolytlösung 14, direkt elektrisch in Verbindung mit der Halbleiterkomponente gebracht. Es kann außerdem an diesem Ende eine zusätzliche Referenzelektrode, z.B. aus Ag/AgCl, vorgesehenen sein um auf diese Weise mit der Ableitelektrode 3 am anderen Ende den unmittelbaren Spannungsabfall an der Insektenantenne 5 zu bestimmen.

Die Antenne (intakter Chemorezeptor) 5 wird mechanisch und elektrisch stabil über eine Antennehaltevorrichtung, beispielsweise bestehend aus einer mit einem viskosen Dichtmaterial 12 abgedichteten Sockelplatte 13, durch die die Antenne zum Flüssig- bzw. Festkontakt durchgeführt werden kann, gehalten. Dabei ist es vorteilhaft möglichst weinig Einzelglieder der Insektenantenne in den Elektrolyten zu tauchen, sodaß ein möglichst langer Bereich der Antenne 5 zur Detektierung der erwünschten Spurenkomponente der in an die Antenne 5 vorbeiströmenden Meßluft eingesetzt wird. Die Sockelplatte 13 ist direkt und mechanisch stabil mit der Wandung 15 verbunden, die ihrerseits über eine Dichtung 16, z.B. O-Ring gedichtet, direkt mit der Halbleiterkomponente verbunden ist.

Die Halbleiterkomponente, vorzugsweise ein FET-Bauelement setzt sich aus einem Grundsubstrat 21 aus z.B. n- bzw. p-dotiertes Silicium zusammen. Abhängig von der Dotierung dieses Grundmaterials weisen die taschenförmige, zur Ausbildung von Source und Drain vorgesehenen Substratbereiche 18 und 18a eine entgegengesetzte Dotierung auf. Diese Substratbereiche 18 und 18a sind über eine vorzugsweise metallische Kontaktierung 20 sowie einen rückseitigen Kontakt 22 (z.B. Ti/Al, Ti/Pd/Au o.ä. leitende Materialien) mit einem festen Träger, z.B. mit dem Leiterplattensubstrat 8, elektrisch passiviert und verkapselt, verbunden. Die Metallisierung 20 kann durch eine Isolatorschicht bzw. -schichtfolge 19 aus SiO₂ oder SiO₂/Si₃N₄ oder SiO₂/Al₂O₃ o.ä. vom Grundsubstrat 22 isoliert, schichtförmig aufgebracht sein.

Anstelle der üblichen Gateelektrode beim FET ist bei dieser Anordnung die Kontaktierung des intakten Chemorezeptors mittels der Elektrolytlösung 14, die gleichzeitig die biologische Nährlösung für die Antenne realisiert. Alternativ zum Elektrolyten wären andere Flüssig- oder auch Festkontakte vorstellbar. In der in Figur 1 und Figur 2 dargestellten Anordnung ist ein flüssigkeitsresistiver Gateisolator 17, z.B. aus SiO₂ oder SiO₂/Si₃N₄ oder SiO₂/Al₂O₃ mit einer Schichtdicke von beispielsweise einigen nm, eingesetzt.

Die direkte Kontaktierung des intakten Chemorezeptors mit der Halbleiterkomponente ermöglicht eine deutliche Verringerung der Störanfälligkeit des Biosensorsystems aufgrund der direkten hochohmigen, rauscharmen Signalverstärkung durch den miniaturisierten, als Signalwandler eingesetzten FET. Dieser direkte Kontakt ermöglicht eine schnelle und schonende, d.h. geringfügig in das Hämolymphesystem der Antenne eingreifende, biologische Präparation, die einerseits eine zuverlässige Signalübertragung zum FET leistet und die andererseits den mit Autolysereaktionen und Ionenverteilungsungleichgewichten verbundenen Eingriff in das biologische Funktionssystem so gering wie möglich hält. Soweit die in den Sensor präparierte Antenne verbraucht ist, kann auf relativ einfache Weise eine neue Antenne an der Stelle der alten präpariert werden. Auf diese Weise ist der übrige Aufbau oftmals wiederverwendbar.

Durch die Entwicklung eines vergleichsweise billigen und robusten Biosensorsystems auf der Basis intakter Chemorezeptoren kann die Detektion von brandspezifischen organischen Emissionen mit hoher Selektivität und Empfindlichkeit durchgeführt werden. Die Kombination des Biosensorsystems mit einem Halbleitersensorsystem verbindet die Vorteile der beiden Verfahren, hohe Nachweisempfindlichkeit und geringe Querempfindlichkeit des Biosensorsystems sowie hohe Standzeit und Betriebssicherheit des Halbleitersensorsystems und auf diese Weise eine verbesserte Brandfrüherkennung ermöglichen. Eine Möglichkeit besteht beispielsweise in der Aktivierung des Biosensorsystems erst bei einem Voralarm durch ein parallel dazu vorhandenes Halbleitersensorsystem.

## Patentansprüche

1. Biosensor mit einem Chemorezeptor (5) zur Detektierung einer oder mehrerer Spurenkomponenten eines Brandes, insbesondere eines Schwelbrandes,
**dadurch gekennzeichnet,**
daß eine Insektenantenne als Chemorezeptor (5) mit einer Halbleiterkomponente verbunden ist.

2. Biosensor nach Anspruch 1,
**dadurch gekennzeichnet,**
daß der Chemorezeptor (5) über einen Elektrolyten (14) mit der Halbleiterkomponente verbunden ist.

3. Biosensor nach einem der vorhergehenden Ansprüche,
**dadurch gekennzeichnet,**
daß der Chemorezeptor (5) räumlich direkt und funktionell mit der Halbleiterkomponente verbunden ist.

4. Biosensor nach einem der vorhergehenden Ansprüche,
**dadurch gekennzeichnet,**
daß eine geringe Zahl an Antennenkettengliedern,
insbesondere ein einziges Antennenkettenglied, an einem Ende des Chemorezeptors (5) direkt oder über den Elektrolyten (14) mit der Halbleiterkomponente verbunden ist.

5. Biosensor nach Anspruch 2 oder 4,
**dadurch gekennzeichnet,**
daß als Elektrolyt (14) ein Fest- oder Flüssigelektrolyt vorgesehen ist.

6. Biosensor nach einem der vorhergehenden Ansprüche,
**dadurch gekennzeichnet,**
daß als Halbleiterkomponente eine auf dem Feldeffekt beruhende Halbleiter-Isolatorstruktur, insbesondere ein Permeable Base Transistor (PBT) oder ein FET vorgesehen ist.

7. Biosensor nach vorhergehendem Anspruch,
**dadurch gekennzeichnet,**
daß der Chemorezeptor (5) mit dieser Halbleiter-Isolatorstruktur so verbunden ist, daß sie die Gate-Elektrode des PBTs bzw. FETs bildet.

8. Biosensor nach einem der vorhergehenden Ansprüche,
**dadurch gekennzeichnet,**
daß der Chemorezeptor (5) an mehreren Stellen der Insektenantenne oder an deren Sinneshaaren mit der Meßelektronik kontaktiert ist und eine weitere Stelle mit der Halbleiterkomponente verbunden ist.

9. Biosensor nach einem der vorhergehenden Ansprüche,
**dadurch gekennzeichnet,**
daß der Chemorezeptor (5) eine Insektenantenne eines Borkenkäfers ist.

## Claims

1. Biosensor having a chemoreceptor (5) for the detection of one or more trace components from a fire, more particularly a smouldering fire,
characterised in that an insect antenna as the chemoreceptor (5) is connected to a semiconductor component.

2. Biosensor according to claim 1,
characterised in that the chemoreceptor (5) is connected to the semiconductor component via an electrolyte (14).

3. Biosensor according to either one of the preceding claims,
characterised in that the chemoreceptor (5) is connected in a spatially direct and functional manner to the semiconductor component.

4. Biosensor according to any of the preceding claims,
characterised in that a small number of antenna chain members, more particularly a single antenna chain member, is connected at one end of the chemoreceptor (5) directly or via the electrolyte (14) to the semiconductor component.

5. Biosensor according to claim 2 or 4,
characterised by the provision of a solid or liquid electrolyte as the electrolyte (15).

6. Biosensor according to any of the preceding claims,
characterised by the provision of a semiconductor insulator structure relying on the field effect, more particularly a permeable base transistor (PBT) or an FET, as the semiconductor component.

7. Biosensor according to the preceding claim,
characterised in that the chemoreceptor (5) is connected to said semiconductor insulator structure in such a way that it forms the gate electrode of the PBT or FET.

8. Biosensor according to any of the preceding claims,
characterised in that the chemoreceptor (5) is caused to contact the measurement electronic system at a plurality of locations on the insect antenna or at sensory hairs thereon, and a further location is connected to the semiconductor component.

9. Biosensor according to any of the preceding claims,
characterised in that the chemoreceptor (5) is an insect antenna of a bark beetle.

## Revendications

1. Biocapteur comportant un chimiorécepteur (5) servant à détecter un ou plusieurs constituants à l'état de tracés d'un feu, notamment d'un feu couvant, caractérisé en ce qu'une antenne d'insecte en tant que chimiorécepteur (5) est reliée à un composant à semiconducteurs.

2. Biocapteur selon la revendication 1, caractérisé en ce que le chimiorécepteur (5) est relié au moyen d'un électrolyte (14) aux composants à semiconducteurs.

3. Biocapteur selon l'une des revendications précédentes, caractérisé en ce que le chimiorécepteur (5) est relié spatialement directement et fonctionnellement au composant à semiconducteurs.

4. Biocapteur selon l'une des revendications précédentes, caractérisé en ce qu'un faible nombre d'éléments de chaîne d'antenne, notamment un seul élément de chaîne d'antenne, au niveau d'une extrémité du chimiorécepteur (5) est relié directement ou par l'intermédiaire de l'électrolyte (14) au composant à semiconducteurs.

5. Biocapteur selon la revendication 2 ou 4, caractérisé en ce qu'on prévoit comme qu'on prévoit comme électrolyte (14) un électrolyte solide ou liquide.

6. Biocapteur selon l'une des revendications précédentes, caractérisé en ce qu'il est prévu comme composant à semiconducteurs, une structure isolante semiconductrice basée sur un effet de champ, notamment un transistor Permeable Base Transistor (PBT) ou un transistor FET.

7. Biocapteur selon la revendication précédente, caractérisé en ce que le chimiorécepteur (5) est relié à cette structure isolante semiconductrice de telle sorte qu'elle forme l'électrode de grille du transistor PBT ou du transistor FET.

8. Biocapteur selon l'une des revendications précédentes, caractérisé en ce que le chimiorécepteur (5) est placé en contact, en plusieurs emplacements de l'antenne d'insecte ou au niveau de ses poils sensitifs, avec le système électronique de mesure et qu'un autre emplacement est relié au composant à semiconducteurs.

9. Biocapteur selon l'une des revendications précédentes, caractérisé en ce que le chimiorécepteur (5) est une antenne de bostryche.
